# EUROPÄISCHE PATENTSCHRIFT

(11) **EP 0 580 619 B1**
(45) Veröffentlichungstag und Bekanntmachung des Hinweises auf die Patenterteilung: **06.09.1995**
(21) Anmeldenummer: 92907052.2
(22) Anmeldetag: 26.03.1992
(51) Int. Cl.: C01F 7/00, C07C 41/03, C07C 53/126, C01B 13/14, C07C 55/02

(54) **VERFAHREN ZUR HERSTELLUNG HYDROPHOBIERTER DOPPELSCHICHTHYDROXID-VERBINDUNGEN**
PROCESS FOR PRODUCING HYDROPHOBISED DOUBLE-LAYER HYDROXIDE COMPOUNDS
PROCEDE DE PREPARATION DE COMPOSES HYDROXYDES HYDROPHOBES A DEUX COUCHES

(30) Priorität: 04.04.1991 DE 4110835
(43) Veröffentlichungstag der Anmeldung: 02.02.1994
(73) Patentinhaber: Henkel Kommanditgesellschaft auf Aktien, 40191 Düsseldorf (DE)
(72) Erfinder: BREUER, Wolfgang, D-4000 Düsseldorf 1 (DE); MAI, Claudia, D-4005 Meerbusch 1 (DE); RATHS, Hans-Christian, D-40789 Monheim (DE); SCHOLZ, Elvira, D-4000 Düsseldorf 13 (DE)
(86) Internationale Anmeldenummer: EP9200675
(87) Internationale Veröffentlichungsnummer: WO9217405

(56) Entgegenhaltungen:
- EP-A- 0 134 936
- EP-A- 0 308 646
- EP-A- 0 339 426
- US-A- 4 351 814

## Beschreibung

Die Erfindung betrifft ein Verfahren zur Herstellung hydrophobierter Doppelschichthydroxid-Verbindungen durch Umsetzung von Doppelschichthydroxid-Verbindungen mit Carbonsäuren oder deren Salzen bei erhöhter Temperatur und anschließender Trocknung der Reaktionsprodukte sowie die Verwendung dieser Stoffe als Katalysatoren in der Alkoxylierung von Verbindungen mit aktiven Wasserstoffatomen oder Fettsäureestern.

Anlagerungsprodukte von Ethylen- und/oder Propylenoxid an primäre Alkohole, die als Alkoholalkoxylate oder Alkoholpolyglycolether bezeichnet werden, besitzen als nichtionische Tenside infolge ihrer ausgezeichneten Detergenseigenschaften und ihrer hohen Kaltwasserlöslichkeit große Bedeutung für die Herstellung von Wasch-, Spül- und Reinigungsmitteln. Im Verlauf der Alkoxylierung, die in der Regel in Gegenwart von leicht löslichen Alkalihydroxiden oder -alkoholaten durchgeführt wird, kommt es jedoch nicht zu einer selektiven Anlagerung einer diskreten Anzahl von Ethylen- und/oder Propylenoxideinheiten an jeweils ein Molekül des Alkohols, die Reaktion folgt vielmehr statistischen Gesetzen und führt zu einem Gemisch homologer Additionsprodukte, deren Alkoxylierungsgrade ein breites Spektrum umfassen.

Aus EP-A-134 936 sind eisenionen-haltige Hydrotalcite bekannt, die einer hydrothermalen Behandlung unterworfen wurden. Carbonsäuren wurden dabei nicht eingesetzt.

Aus US-A-4 351 814 sind Hydrotalcite bekannt, die als Anion Tartrat aufweisen. Es werden bei deren Herstellung keine hydrothermalen Bedingungen angewandt.

Aus **J.Am.Oil.Chem.Soc. 63, 691 (1986)** und **HAPPI 52 (1986)** ist bekannt, daß die Verteilung der Alkoxylierungsgrade im Gemisch der Alkoholalkoxylate, die sogenannte "Homologenverteilung", die Eigenschaften der erhaltenen Additionsprodukte maßgeblich beeinflußt. Dabei wurde gefunden, daß Produkte mit "eingeengter" Homologenverteilung, sogenannte "narrow-range alkoxylates", Vorteile gegenüber vergleichbaren Produkten mit "breiter" Homologenverteilung aufweisen, so z. B.:
- niedrigere Fließpunkte,
- höhere Rauchpunkte,
- geringere Anzahl von Molen Alkylenoxid zum Erreichen der Wasserlöslichkeit,
- geringere Anteile an nichtumgesetzten Alkohol und damit verbunden, eine verminderte Geruchsbelastung sowie
- Reduzierung des Plumings beim Sprühtrocknen von Alkoholalkoxylat-enthaltenden Waschmittelslurries.

In der Vergangenheit hat es nicht an Versuchen gemangelt, durch Variation des Alkoxylierungskatalysators, Anlagerungsprodukte von Ethylen- und/oder Propylenoxid an primäre Alkohole herzustellen, die eine eingeengte Homologenverteilung aufweisen und über das geschilderte Eigenschaftsprofil verfügen.

Als besonders effektive heterogene, d. h. nicht im Reaktionsgemisch lösliche Katalysatoren zur Herstellung von nichtionischen Tensiden mit eingeengter Homologenverteilung haben sich Doppelschichthydroxid-Verbindungen, beispielsweise vom Typ der Hydrotalcite bewährt.

So beschreiben die Deutsche Patentanmeldung **DE 38 43 713 A1** sowie die US-Patentschrift **US 4,962,237** Verfahren zur Alkoxylierung von Verbindungen mit aktiven Wasserstoffatomen, die in Gegenwart von Hydrotalciten durchgeführt werden. Die Hydrotalcite müssen jedoch zuvor durch mehrstündiges Erhitzen auf Temperaturen von 400 bis 600°C in eine für katalytische Zwecke geeignete, calcinierte Form überführt werden. Obschon die Einengung der Homologenverteilung der nach diesem Verfahren zugänglichen nichtionischen Tenside als zufriedenstellend angesehen werden kann, ist die Herstellung des Katalysators, insbesondere im Hinblick auf die Calcinierung, mit einem hohen Aufwand an Energie und Zeit verbunden und daher unvorteilhaft. Weiterhin sind calcinierte Verbindungen anfällig gegen Wasserspuren und das Kohlendioxid der Luft (Rückreaktion der Calcinierung), so daß ihr Anwendungsbereich und ihre Lagerstabilität infolge des Aktivitätsverlustes begrenzt sind.

Die Aufgabe der Erfindung bestand somit darin, ein Verfahren zur Herstellung aktivierter Doppelschichthydroxid-Verbindungen zu entwickeln, das frei von den geschilderten Nachteilen ist.

Gegenstand der Erfindung ist ein Verfahren zur Herstellung von hydrophobierten Doppelschichthydroxid-Verbindungen, das sich dadurch auszeichnet, daß man Doppelschichthydroxid-Verbindungen der allgemeinen Formel **(I)**,

**[M**^{**(II)**}_{**1-x**}**M**^{**(III)**}_{**x**}**(OH)₂] A**_{**x**} *** n H₂O (I)**

in der
- M^{(II)}: ein 2-wertiges Metallkation,
- M^{(III)}: ein 3-wertiges Metallkation,
- A: ein Äquivalent einer ein- und/oder mehrbasischen anorganischen Säure,
- x: eine Zahl von 0,2 bis 0,5 sowie
- n: eine Zahl von 0 bis 10
bedeuten, bei erhöhter Temperatur und unter autogenem Druck mit
a) mindestens einer aliphatischen Monocarbonsäure mit 2 bis 24 Kohlenstoffatomen und/oder
b) mindestens einer aliphatischen Dicarbonsäure mit 4 bis 48 Kohlenstoffatomen oder deren Salz
umsetzt und das Reaktionsprodukt anschließend trocknet.

Überraschenderweise wurde gefunden, daß die Hydrophobierung von Doppelschichthydroxiden im Sinne des erfindungsgemäßen Verfahrens, das heißt, die Umsetzung der Schichtverbindungen mit organischen Säuren und anschließende Trocknung, in gleicher Weise zu einer Aktivierung führt, wie dies nach dem Stand der Technik bislang nur durch Calcinierung zu erreichen gewesen ist. Während die Calcinierung Temperaturen von mindestens 400°C erfordert, kommt das erfindungsgemäßen Verfahrens mit Temperaturen von maximal 250°C aus und stellt somit im Hinblick auf die Energiebilanz eine deutliche Verbesserung dar.

Unter Doppelschichthydroxid-Verbindungen sind Polykationen mit innerkristallinem Ladungsausgleich durch bewegliche Zwischenschichtanionen zu verstehen [**Chimia 24, 99 (1970)**].

Als 2-wertige Metallionen gemäß Formel **(I)** kommen Kationen in Betracht, die ausgewählt sind aus der Gruppe, die von Magnesium, Zink, Calcium, Eisen, Cobalt, Cupfer, Cadmium, Nickel und Mangan gebildet wird. Bevorzugt werden solche Doppelschichthydroxid-Verbindungen eingesetzt, bei denen M^{(II)} für Magnesium steht.

Unter 3-wertigen Metallionen gemäß Formel **(I)** sind Kationen zu verstehen, die ausgewählt sind aus der Gruppe, die von Aluminium, Eisen, Chrom, Mangan, Wismut und Cer gebildet wird. Bevorzugt werden solche Doppelschichthydroxid-Verbindungen eingesetzt, bei denen M^{(III)} für Aluminium steht.

Gemäß Formel **(I)** steht A für Anionen ausgewählt aus der Gruppe, die von Carbonat, Hydrogencarbonat, Sulfat, Nitrit, Nitrat, Phosphat, Hydroxid und Halogeniden gebildet wird. Bevorzugt werden solche Doppelschichthydroxid-Verbindungen eingesetzt, bei denen A für Carbonat steht.

Bevorzugtes Ausgangsmaterial für die Herstellung der hydrophobierten Doppelschichthydroxid-Verbindungen ist im Sinne des erfindungsgemäßen Verfahrens Hydrotalcit, ein Stoff, der sowohl als Mineral in der Natur vorkommt, als auch nach einschlägigen Verfahren auf synthetischem Wege erhalten werden kann. In diesem Zusammenhang sei auf die Druckschriften **DE 15 92 126 C, DE 33 46 943 A1, DE 33 06 822 A1** und **EP 0 207 811 A1** verwiesen.

Hydrotalcit weist im Idealfall die Formel **(II)**

**[Mg₆Al₂(OH)₁₆]CO₃ * 4 H₂O (II)**

auf, dessen Struktur von derjenigen des Brucits [Mg(OH)₂] abgeleitet ist. Brucit kristallisiert in einer Schichtstruktur mit den Metallionen in Oktaederlücken zwischen zwei Schichten aus dichtgepackten Hydroxylionen, wobei nur jede zweite Schicht der Oktaederlücken besetzt ist. Im Hydrotalcit sind einige Magnesiumionen durch Aluminiumionen ersetzt, wodurch das Schichtpaket eine positive Ladung erhält. Diese wird durch die Anionen ausgeglichen, die sich zusammen mit Kristallwasser in den Zwischenschichten befinden.

Weitere Doppelschichthydroxid-Verbindungen, die als Ausgangsstoffe für die Herstellung der hydrophobierten Species in Betracht kommen, sind beispielsweise:
- Magaldrat [Mg₁₀Al₅(OH)₃₀](SO₄)₂OH * n H₂O
- Pyroaurit [Mg₆Fe₂(OH)₁₆]CO₃ * 4,5 H₂O
- Hydrocalumit [Ca₂Al(OH)₆]NO₃ * n H₂O
Die Hydrophobierung erfolgt durch Umsetzung der Doppelschichthydroxid-Verbindungen mit aliphatischen Mono- oder Dicarbonsäuren oder deren Salze.

**Monocarbonsäuren.** Unter Monocarbonsäuren, die sich zur Hydrophobierung der Doppelschichthydroxide eignen, sind aliphatische Carbonsäuren mit 2 bis 24 Kohlenstoffatomen zu verstehen. Typische Beispiele sind Essigsäure, Propionsäure, Buttersäure, Valeriansäure, Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Chaulmoograsäure, Ricinolsäure, Linolsäure, Linolensäure, Arachinsäure, Gadoleinsäure, Behensäure, Erucasäure, Arachidonsäure oder Clupanodonsäure. Bevorzugt sind Monocarbonsäuren mit 12 bis 18 Kohlenstoffatomen, insbesondere Laurinsäure sowie deren Natriumsalze.

Wie in der Fettchemie üblich, kommen als Monocarbonsäuren auch technische Schnitte in Betracht, wie sie bei der Druckspaltung von natürlichen Fetten und Ölen, beispielsweise Kokosöl, Palmöl, Palmkernöl, Baumwollsaatöl, Erdnußöl, Sojaöl, Rüböl, Sonnenblumenöl, Leinöl, Korianderöl, Ricinusöl, Rindertalg oder Fischöl anfallen. Bevorzugt sind technische C_{12/18}- beziehungsweise C_{12/14}-Fettsäuregemische auf Basis von Kokosöl sowie deren Natriumsalze.

**Dicarbonsäuren.** Die Hydrophobierung kann auch mit Dicarbonsäuren mit 4 bis 48 Kohlenstoffatomen durchgeführt werden. Typische Beispiele sind Bernsteinsäure, Maleinsäure, Fumarsäure, Adipinsäure, Pimelinsäure, Korksäure oder Sebacinsäure. Weiterhin kommen auch sogenannte Dimerfettsäuren in Betracht, die beispielsweise durch Erhitzen von ungesättigten Fettsäuren in Gegenwart von Montmorilloniten gewonnen werden und bis zu 48 Kohlenstoffatome enthalten können.

Im Sinne der vorliegenden Erfindung werden hydrophobierte Doppelschichthydroxide angestrebt, bei denen möglichst alle ein- oder mehrbasischen Anionen A in den Verbindungen der Formel **(I)** gegen organische Säurereste ausgetauscht sind. Aus diesem Grund werden die Doppelschichthydroxid-Verbindungen der Formel **(I)** mit den Carbonsäuren oder derem Salzen in solchen Mengen umsetzt, daß hydrophobierte Doppelschichthydroxid-Verbindungen mit einem Carboxylat-Anteil von 15 bis 70, vorzugsweise 15 bis 50 Gew.-% - bezogen auf das Gesamtgewicht - erhalten werden.

Hierzu empfiehlt es sich, die Doppelschichthydroxid-Verbindungen mit den Monocarbonsäuren im molaren Verhältnis von 6 : 1 bis 1 : 10, insbesondere 3 : 1 bis 1 : 3 beziehungsweise mit den Dicarbonsäuren im molaren Verhältnis von 3 : 1 bis 1 : 5, insbesondere 1,5 : 1 bis 1 : 3 umzusetzen. Das Molverhältnis ist dabei auf die beim Austausch zu Verfügung stehenden Anionen A der jeweilig eingesetzten Doppelschichthydroxid-Verbindungen einzustellen.

Zur Durchführung des erfindungsgemäßen Verfahren ist es ausreichend, die beiden Komponenten - Doppelschichthydroxidverbindung und Carbonsäure - gegebenenfalls in Wasser suspendiert in einem Druckgefäß vorzulegen und unter autogenem Druck über eine Zeitspanne von 0,1 bis 5, vorzugsweise 0,5 bis 2 h auf Temperaturen von 100 bis 250, vorzugsweise 150 bis 220°C zu erhitzen. Es hat sich als vorteilhaft erwiesen, die bei der Hydrophobierung freiwerdenden Gase - Wasserdampf und Kohlendioxid - durch teilweises Entspannen des Autoklaven abzulassen.

Das rohe hydrophobierte Produkt, das noch eine Restfeuchte aufweist, wird anschließend einer Trocknung unterworfen. Diese kann beispielsweise im Trockenschrank, gegebenenfalls unter vermindertem Druck, bei Temperaturen von 100 bis 220°C durchgeführt werden.

Anhand von Röntgenbeugungsdiagrammen konnte gezeigt werden, daß in den hydrophobierten Doppelschichthydroxid-Verbindungen die Schichtstruktur unter Aufweitung der Schichtabstände erhalten geblieben ist.

Die nach dem erfindungsgemäßen Verfahren erhältlichen hydrophobierten Doppelschichthydroxid-Verbindungen katalysieren die Anlagerung von Alkylenoxiden an Verbindungen mit aktiven Wasserstoffatomen oder Fettsäureester.

Ein weiterer Gegenstand der Erfindung betrifft daher die Verwendung dieser hydrophobierten Doppelschichthydroxid-Verbindungen als Katalysatoren in der Alkoxylierung von Verbindungen mit aktiven Wasserstoffatomen oder von Fettsäureester.

Die Verwendung der erfindungsgemäß hergestellten hydrophobierten Doppelschichthydroxid-Verbindungen erlaubt die Alkoxylierung von Verbindungen mit aktiven Wasserstoffatomen sowie von Fettsäureestern in kurzen Reaktionszeiten und hohen Ausbeuten. Die Reaktionsprodukte weisen dabei eine vorteilhafte eingeengte Homologenverteilung auf, wobei die Verteilungskurve der idealen Poisson-Verteilung nahe kommt. Die Reaktionsprodukte zeichnen sich darüber hinaus durch geringe Gehalte an nichtumgesetzten Ausgangsstoffen sowie Dioxan aus. Die erfindungsgemäß hergestellten hydrophobierten Doppelschichthydroxid-Verbindungen können leicht in das Reaktionsgemisch eingearbeitet werden. Da sie sich in der Mischung nicht lösen, ist ihre Abtrennung beispielsweise durch Filtration unkompliziert. Sie können jedoch auch in der Reaktionsmischung verbleiben, sofern ihre Anwesenheit die Weiterverwendung der Produkte nicht beeinträchtigt.

Beispiele für Verbindungen mit aktiven Wasserstoffen, die in Gegenwart der nach dem erfindungsgemäßen Verfahren erhältlichen hydrophobierten Doppelschichthydroxid-Verbindungen alkoxyliert werden können, sind Fettsäuren, Hydroxyfettsäuren, Fettsäureamide, Fettalkohole, Alkylphenole, Polyglycole, Fettamine, Fettsäurealkanolamide oder vicinal hydroxy- bzw. alkoxy-substituierte Alkane.

Bei der Alkoxylierung handelt es sich um ein an sich bekanntes großtechnisches Verfahren, das bei Temperaturen von 120 bis 220, vorzugsweise 150 bis 190°C und Drücken von 1 bis 6 bar durchgeführt wird. Die hydrophobierten Doppelschichthydroxid-Verbindungen können dabei in Mengen von 0,1 bis 3 Gew.-% - bezogen auf das Endprodukt der Alkoxylierung - eingesetzt werden.

Im Hinblick auf Aktivität und Einengung der Homologenverteilung hat es sich als optimal erwiesen, als hydrophobierte Doppelschichthydroxidverbindung einen mit Laurinsäure hydrophobierten Hydrotalcit der Formel **(II)** zu verwenden.

Die folgenden Beispiele sollen den Gegenstand der Erfindung näher erläutern, ohne ihn darauf einzuschränken.

### Beispiele

### Beispiel 1:

**Herstellung eines hydrophobierten Hydrotalcit-Katalysators.** In einem Autoklaven wurde ein Gemisch aus 50 g (0,08 mol) Hydrotalcit (handelsübliche Qualität) und 32 g (0,16 mol) Laurinsäure vorgelegt und in 500 ml Wasser suspendiert. Die Reaktionsmischung wurde über einen Zeitraum von 60 min unter einem autogenen Druck von 21 bar auf 200°C erhitzt. In Abständen von ca. 15 min wurden die aus dem Ansatz freigesetzten Gase - Kohlendioxid und Wasserdampf - durch Entspannen bis auf einen Druck von 10 bar abgelassen. Nach dem Reaktionsende wurde die Reaktionsmischung abgekühlt, der hydrophobierte Hydrotalcit abfiltriert und anschließend im Trockenschrank zunächst bei 110°C und dann bei 200°C getrocknet. Die Menge an wasserfreiem hydrophobiertem Hydrotalcit betrug ca. 70 g.

| | | |
|---|---|---|
| Zusammensetzung: | Mg | 14,9 Gew.-% |
| | Al | 5,9 Gew.-% |
| | C | 33,1 Gew.-% |
| Verhältnis Mg/Al | | 2,80 : 1,00 |
| Verhältnis Mg/Laurat | | 0,95 : 1,00 |

### Beispiel 2:

Beispiel 1 wurde wiederholt. Die Umsetzung von Hydrotalcit und Laurinsäure wurde jedoch über einen Zeitraum von 2 h durchgeführt.

| | | |
|---|---|---|
| Zusammensetzung: | Mg | 13,8 Gew.-% |
| | Al | 5,4 Gew.-% |
| | C | 30,8 Gew.-% |
| Verhältnis Mg/Al | | 2,84 : 1,00 |
| Verhältnis Mg/Laurat | | 0,94 : 1,00 |

### Vergleichsbeispiel 1:

20 g (0,033 mol) eines handelsüblichen Hydrotalcits, der zuvor über einen Zeitraum von 2h bei einer Temperatur von 550°C calciniert worden war, wurden in 200 ml Wasser suspendiert und mit einer Lösung von 25 g (0,125 mol) Laurinsäure in 100 ml Isopropylalkohol versetzt. Die Reaktionsmischung wurde 3 h lang auf 70°C erwärmt, abgekühlt und abfiltriert. Nach dem Waschen des Filterkuchens mit Isopropylalkohol wurde dieser bei 100°C und 100 hPa bis zur Massenkonstanz getrocknet. Der hydrophobierte Hydrotalcit wurde in in Form eines farblosen Pulvers in einer Menge von ca. 42 g erhalten. Der Lauratgehalt betrug ca. 44 Gew.-%.

### Beispiel 3:

In einem Autoklaven wurden 2,5 g des Katalysators gemäß Beispiel 1 in 300 g (1,55 mol) C_{12/14}-Kokosfettalkohol (Lorol^{(R)} S, Hydroxylzahl = 290, Fa.Henkel KGaA) suspendiert. Der Reaktor wurde mit Stickstoff gespült und 30 min lang bei 100°C evakuiert.

Anschließend wurde die Temperatur auf 150°C gesteigert und über einen Zeitraum von 20 min portionsweise 204 g (4,6 mol) Ethylenoxid bis zu einem Druck von 6 bar aufgepreßt, wobei die Temperatur bis auf 180°C anstieg. Im Anschluß an eine Nachreaktion von 30 min wurde das Reaktionsgemisch abgekühlt, entspannt und der Katalysator abfiltriert. Die Homologenverteilung des resultierenden Fettalkholpolyethylenglycolethers ist in Abb.1 wiedergegeben.

In Abb.1 ist neben der Homologenverteilung des Produktes gemäß Beispiel 1 zum Vergleich auch die Homologenverteilung für ein entsprechendes Produkt angegeben, das in Gegenwart von Natriummethylat als Alkoxylierungskatalysator hergestellt wurde. Es zeigt sich, daß bei erfindungsgemäßer Verwendung der hydrophobierten Doppelschichthydroxid-Verbindungen eine vorteilhafte eingeengte Homologenverteilung resultiert.

## Patentansprüche

1. Verfahren zur Herstellung von hydrophobierten Doppelschichthydroxid-Verbindungen, **dadurch gekennzeichnet,** daß man Doppelschichthydroxid-Verbindungen der allgemeinen Formel **(I)**,
**[M**^{**(II)**}_{**1-x**}**M**^{**(III)**}_{**x**}**(OH)₂] A**_{**x**} *** n H₂O (I)**
in der
M^{(II)} ein 2-wertiges Metallkation,
M^{(III)} ein 3-wertiges Metallkation,
A ein Äquivalent einer ein- und/oder mehrbasischen anorganischen Säure,
x eine Zahl von 0,2 bis 0,5 sowie
n eine Zahl von 0 bis 10
bedeuten,
bei erhöhter Temperatur und unter autogenem Druck mit
a) mindestens einer aliphatischen Carbonsäure mit 2 bis 24 Kohlenstoffatomen und/oder
b) mindestens einer aliphatischen Dicarbonsäure mit 4 bis 48 Kohlenstoffatomen
umsetzt und das Reaktionsprodukt anschließend trocknet.

2. Verfahren nach Anspruch 1, **dadurch gekennzeichnet,** daß man Doppelschichthydroxid-Verbindungen der Formel **(I)** einsetzt, in der M^{(II)} für ein zweiwertiges Metallkation ausgewählt aus der Gruppe, die von Magnesium, Zink, Calcium, Eisen, Cobalt, Cupfer, Cadmium, Nickel und Mangan gebildet wird, steht.

3. Verfahren nach den Ansprüchen 1 und 2, **dadurch gekennzeichnet,** daß man Doppelschichthydroxid-Verbindungen der Formel **(I)** einsetzt, in der M^{(III)} für ein dreiwertiges Metallkation ausgewählt aus der Gruppe, die von Aluminium, Eisen, Chrom, Mangan, Wismut und Cer gebildet wird, steht.

4. Verfahren nach mindestens einem der Ansprüche 1 bis 3, **dadurch gekennzeichnet,** daß man Doppelschichthydroxid-Verbindungen der Formel **(I)** einsetzt, in der A für ein Anion ausgewählt aus der Gruppe, die von Carbonat, Hydrogencarbonat, Sulfat, Nitrit, Nitrat, Phosphat, Hydroxid und Halogeniden gebildet wird, steht.

5. Verfahren nach mindestens einem der Ansprüche 1 bis 4, **dadurch gekennzeichnet,** daß man als Carbonsäuren Fettsäuren mit 12 bis 18 Kohlenstoffatomen einsetzt.

6. Verfahren nach mindestens einem der Ansprüche 1 bis 5 **dadurch gekennzeichnet,** daß man die Doppelschichthydroxid-Verbindungen der Formel **(I)** mit den Carbonsäuren in solchen Mengen umsetzt, daß hydrophobierte Doppelschichthydroxid-Verbindungen mit einem Carboxylat-Anteil von 15 bis 70 Gew.-% - bezogen auf das Gesamtgewicht - erhalten werden.

7. Verfahren nach mindestens einem der Ansprüche 1 bis 6, **dadurch gekennzeichnet,** daß man die Umsetzung der DoppelschichthydroxidVerbindungen mit den Carbonsäuren bei Temperaturen von 100 bis 250°C durchführt.

8. Verfahren nach mindestens einem der Ansprüche 1 bis 7, **dadurch gekennzeichnet,** daß man die Trocknung bei Temperaturen von 100 bis 220°C, gegebenenfalls unter vermindertem Druck durchführt.

9. Verwendung von hydrophobierten Doppelschichthydroxid-Verbindungen erhältlich nach dem Verfahren nach mindestens einem der Ansprüche 1 bis 8 als Katalysatoren in der Alkoxylierung von Verbindungen mit aktiven Wasserstoffatomen oder Fettsäureestern.

10. Verwendung nach Anspruch 9, **dadurch gekennzeichnet,** daß die Verbindungen mit aktiven Wasserstoffatomen ausgewählt sind aus der Gruppe, die von Fettsäuren, Hydroxyfettsäuren, Fettsäureamiden, Fettalkoholen, Alkylphenolen, Polyglycolen, Fettaminen, Fettsäurealkanolamiden oder vicinal hydroxy- bzw. alkoxy- substituierten Alkanen gebildet wird.

11. Verwendung nach den Ansprüchen 9 und 10, **dadurch gekennzeichnet,** daß man die hydrophobierten Doppelschichthydroxid-Verbindungen in Mengen von 0,1 bis 3 Gew.-% - bezogen auf das Endprodukt der Alkoxylierung - einsetzt.

12. Verwendung nach mindestens einem der Ansprüche 9 bis 11, **dadurch gekennzeichnet,** daß man als hydrophobierte Doppelschichthydroxid-Verbindung einen mit Laurinsäure hydrophobierten Hydrotalcit einsetzt.

## Claims

1. A process for the production of hydrophobicized double layer hydroxide compounds, characterized in that double layer hydroxide compounds corresponding to general formula (I):
[M^{(II)}₁₋ₓM^{(III)}ₓ(OH)₂] Aₓ · n H₂O (I)
in which
M^{(II)} is a divalent metal cation,
M^{(III)} is a trivalent metal cation,
A is an equivalent of a monobasic and/or polybasic inorganic acid,
x is a number of 0.2 to 0.5 and
n is a number of 0 to 10,
are reacted at elevated temperature and under autogenous pressure with
a) at least one aliphatic carboxylic acid containing 2 to 24 carbon atoms and/or
b) at least one aliphatic dicarboxylic acid containing 4 to 48 carbon atoms,
and the reaction product is subsequently dried.

2. A process as claimed in claim 1, characterized in that double layer hydroxide compounds corresponding to formula (I), in which M^{(II)} is a divalent metal cation selected from the group consisting of magnesium, zinc, calcium, iron, cobalt, copper, cadmium, nickel and manganese, are used.

3. A process as claimed in claims 1 and 3, characterized in that double layer hydroxide compounds corresponding to formula (I), in which M^{(III)} is a trivalent metal cation selected from the group consisting of aluminium, iron, chromium, manganese, bismuth and cerium, are used.

4. A process as claimed in at least one of claims 1 to 3, characterized in that double layer hydroxide compounds corresponding to formula (I), in which A is an anion selected from the group consisting of carbonate, hydrogen carbonate, sulfate, nitrite, nitrate, phosphate, hydroxide and halides, are used.

5. A process as claimed in at least one of claims 1 to 4, characterized in that C₁₂₋₁₈ fatty acids are used as the carboxylic acids.

6. A process as claimed in at least one of claims 1 to 5, characterized in that the double layer hydroxide compounds corresponding to formula (I) are reacted with the carboxylic acids in such quantities that hydrophobicized double layer hydroxide compounds containing 15 to 70% by weight of carboxylate, based on their total weight, are obtained.

7. A process as claimed in at least one of claims 1 to 6, characterized in that the reaction of the double layer hydroxide compounds with the carboxylic acids is carried out at temperatures of 100 to 250°C.

8. A process as claimed in at least one of claims 1 to 7, characterized in that the drying step is carried out at temperatures of 100 to 220°C, optionally under reduced pressure.

9. The use of the hydrophobicized double layer hydroxide compounds obtainable by the process claimed in at least one of claims 1 to 8 as catalysts in the alkoxylation of compounds containing active hydrogen atoms or fatty acid esters.

10. The use claimed in claim 9, characterized in that the compounds containing active hydrogen atoms are selected from the group consisting of fatty acids, hydroxyfatty acids, fatty acid amides, fatty alcohols, alkylphenols, polyglycols, fatty amines, fatty acid alkanolamides or vicinally hydroxy- or alkoxy-substituted alkanes.

11. The use claimed in claims 9 and 10, characterized in that the hydrophobicized double layer hydroxide compounds are used in quantities of 0.1 to 3% by weight, based on the end product of the alkoxylation reaction.

12. The use claimed in at least one of claims 9 to 11, characterized in that a hydrotalcite hydrophobicized with lauric acid is used as the hydrophobicized double layer hydroxide compound.

## Revendications

1. Procédé d'obtention de composés hydroxydés à double couche rendus hydrophobes, caractérisé en ce que l'on fait réagir des composés hydroxydés à double couche de formule générale I.
[M^{(II)}₁₋ₓM^{(III)}ₓ(OH)₂]Aₓ * n H₂O (I)
dans laquelle :
M^{(II)} signifie un cation métallique bivalent,
M^{(III)} signifie un cation métallique trivalent,
A signifie un équivalent d'acide minéral uni et/ou pluribasique,
x signifie un nombre allant de 0,2 à 0,5 et,
n signifie un nombre allant de 0 à 10
à température élevée et sous pression autogène avec,
a) au moins un acide monocarboxylique aliphatique ayant de 2 à 24 atomes de carbone et/ou
b) au moins un acide dicarboxylique aliphatique ayant de 4 à 48 atomes de carbone,
et en ce que l'on sèche ensuite le produit réactionnel

2. Procédé selon la revendication 1, caractérisé en ce que l'on met en oeuvre des composés hydroxydés à double couche, de formule (I), dans laquelle M^{(II)} représente un cation métallique divalent choisi dans le groupe, qui est formé du magnésium, du zinc, du calcium, du fer, du cobalt, du cuivre, du cadmium, du nickel, et du manganèse.

3. Procédé selon les revendications 1 et 2, caractérisé en ce que l'on met en oeuvre des composés hydroxydés à double couche, de formule (I), dans laquelle M^{(III)} représente un cation métallique trivalent choisi dans le groupe, qui est formé de l'aluminium, du fer, du chrome, du manganèse, du bismuth et du cérium.

4. Procédé selon au moins l'une des revendications 1 à 3, caractérisé en ce que l'on met en oeuvre des composés hydroxydés à double couche de formule (I), dans laquelle A représente un anion choisi dans le groupe, qui est formé par un carbonate, un hydrogenocarbonate, un sulfate, un nitrite, un nitrate, un phosphate, un hydroxyde et des halogénures.

5. Procédé selon au moins l'une des revendications 1 à 4, caractérisé en ce que l'on met en oeuvre comme acides carboxyliques, des acides gras ayant de 12 à 18 atomes de carbone.

6. Procédé selon au moins l'une des revendications 1 à 5, caractérisé en ce que l'on fait réagir les composés hydroxydés à double couche de formule (I) avec les acides carboxyliques en quantités telles, que l'on obtient des composés hydroxydes à double couche rendus hydrophobes avec une proportion de carboxylate allant de 15 à 70 % en poids, rapporté au poids total.

7. Procédé selon au moins l'une des revendications 1 à 6, caractérisé en ce que l'on effectue la réaction des composés hydroxydés à double couche avec les acides carboxyliques à des températures allant de 100 à 250°C.

8. Procédé selon au moins l'une des revendications 1 à 7, caractérisé en ce que l'on effectue le séchage à des températures allant de 100 à 220°C, le cas échéant sous pression réduite.

9. Utilisation des composés hydroxydés à double couche rendus hydrophobes accessibles selon le procédé selon au moins une des revendications 1 à 8, comme catalyseurs dans l'alcoxylation de composés ayant des atomes d'hydrogène actifs ou d'esters d'acide gras.

10. Utilisation selon la revendication 9, caractérisée en ce que les composés ayant des atomes d'hydrogène actifs sont choisis dans le groupe formé des acides gras, des acides gras hydroxylés, des amides d'acide gras, des alcools gras, des alcoylphénols, des polyglycols, des amines grasses, des alcanolamides d'acides gras, et des alcanes substitués par des hydroxy ou des alcoxy vicinaux.

11. Utilisation selon les revendications 9 et 10, caractérisée en ce que l'on met en oeuvre les composés hydroxydés à double couche rendus hydrophobes en quantités allant de 0,1 à 3 % en poids, rapportées au produit final de l'alcoxylation.

12. Utilisation selon au moins l'une des revendications 9 à 11, caractérisée en ce que l'on met en oeuvre comme composé hydroxydé à double couche rendu hydrophobe, une hydrotalcite rendue hydrophobe avec de l'acide laurique.
